# DEMANDE DE BREVET EUROPEEN

(11) **EP 2 409 694 A1**
(43) Date de publication de la demande: **25.01.2012**
(21) Numéro de dépôt: 11008255.9
(22) Date de dépôt: 12.02.2009
(51) Int. Cl.: A61K 31/165, A61K 31/55, A61P 9/10, A61P 9/04

(54) **Association d'un inhibiteur du courant If sinusal et d'un bêta-bloquant.**

(30) Priorité: 14.02.2008 FR 0800800
(62) Demande divisionnaire de: 09290099.2
(71) Demandeur: Les Laboratoires Servier, 92284 Suresnes Cedex (FR)
(72) Inventeur: Lerebours-Pigeonnière, Guy, 92300 Levallois-Perret (FR); Calvet, Jean-Henri, 75003 Paris (FR)
(74) Mandataire: Bestel, Delphine

(57) **Abrégé**

Association d'un inhibiteur du courant If sinusal et d'un β-bloquant, ainsi que les compositions pharmaceutiques qui la contiennent.

## Description

La présente invention concerne l'association fixe d'un inhibiteur du courant If sinusal et d'un bêta-bloquant ou β-bloquant, ainsi que les compositions pharmaceutiques qui les contiennent.

Plus particulièrement, la présente invention concerne une association fixe d'un inhibiteur du courant If sinusal qui est l'ivabradine ou 3-{3-[{[(7S)-5,4-diméthoxybicyclo[4.2.0]octa-1,3,5-trién-7-yl]méthyl}(méthyl)amino]propyl}-7,B-di-méthoxy-1,3,4,5-tétrahydro-2H-3-benzazépin-2-one de formule (I) : 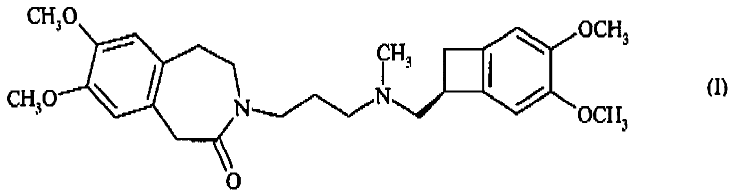 ainsi que ses hydrates, formes cristallines et sels d'addition à un acide pharmaceutiquement acceptable, et d'un β-bloquant,

De manière préférée, la présente invention a pour objet une association fixe entre un β-bloquant de type cardio-sélectif et plus particulièrement l'atenolol ou un de ses hydrates, formes cristallines ou sels d'addition à un acide pharmaceutiquement acceptable, et l'ivabradine chlorydrate ou un de ses hydrates ou formes cristallines en tant qu'inhibiteur du courant If sinusal.

Les inhibiteurs du courant If sinusal et plus particulièrement l'ivabradine, ainsi que ses hydrates, formes cristallines et ses sels d'addition à un acide pharmaceutiquement acceptable, et plus particulièrement son chlorhydrate, possèdent des propriétés pharmacologiques et thérapeutiques très intéressantes, notamment des propriétés chronotropes négatives (réduction de la fréquence cardiaque), qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0534 859.

Les β-bloquants ou β-bloqueurs sont des molécules inhibant les récepteurs adrénergiques β. Les β-bloquant ont pour effet de ralentir le coeur en diminuant la pente de dépolarisation, de diminuer le travail du coeur et de réduire ses besoins en oxygène. En conséquence, les β-bloquants sont utilisés dans le traitement de l'angor, en particulier en prévention des crises. De manière paradoxale en apparence, les β-bloquants sont utilisés dans le traitement de l'insuffisance cardiaque.

De manière préférée, les β-bloquants selon l'invention sont des β-bloquants cardio-sélectifs c'est à dire bloquant préférentiellement les récepteurs β1-cardiaques. Le caractère cardio-séfectif permet de diminuer les effets secondaires en particulier la vasoconstriction périphérique et la bronchoconstriction,
Les β-bloquants cardio-sélectifs sont sélectionnés plus particulièrement dans la liste suivante : acébutolol, aténolol, bétaxolol, bisoprolol, esmolol, metoprolol et nébivolol.

La littérature et les leaders d'opinion dans le domaine des maladies cardiovasculaires concluent d'une manière générale à la difficulté de la démonstration de la supériorité des associations β-bloquant plus une autre classe de composés anti-angor versus un β-bloquant en monothérapie dans le traitement de l'angor stable.

La présente invention met en évidence de manière surprenante que les inhibiteurs du courant If sinusal et plus particulièrement l'ivabradine est capable de potentialiser les effets des β-bloquants sur l'augmentation de la capacité à l'exercice. Cette augmentation de la capacité à l'exercice est donc liée à une synergie entre les principes actifs, i.e. un inhibiteur du courant If sinusal et un β-bloquant.

Dans les compositions pharmaceutiques selon l'invention, les quantités d'inhibiteur du courant If sinusal et de β-bloquant sont adaptées à la nature de ces principes actifs et leurs proportions relatives sont donc variables en fonction desdits principes actifs.

Les compositions pharmaceutiques selon l'invention présentent des proportions comprises entre 1,6% et 80% de la masse totale des principes actifs pour l'inhibiteur du courant If sinusal et entre 20% et 98% de la masse totale des principes actifs pour le β-bloquant.

Lorsque l'inhibiteur du courant If sinusal est l'ivabradine sous forme de sel de chlorydrate et que le β-bloquant est l'atenolol les pourcentages préférés pour cette association sont autour de 25% d'ivabradine sous forme de sel de chlorydrate contre 75% d'atenolol.

La présente invention concerne également les compositions pharmaceutiques comprenant comme principe actif une association d un inhibiteur du courant If sinusal et d'un β-bloquant, éventuellement sous forme de sels pharmaceutiquement acceptables, seuls ou avec un ou plusieurs véhicules ou excipients inertes, non toxiques et appropriés.

Dans les compositions pharmaceutiques selon l'invention, la fraction massique en principes actifs (masse des principes actifs sur la masse totale de la composition) est avantageusement comprise entre 5 et 50%.

En ce qui concerne les excipients pharmaceutiquement acceptables, on peut citer à titre non limitatif les liants, les diluants, les délitants, les stabilisants, les conservateurs, les lubrifiants, les odorants, les aromatisants ou les édulcorants.

A titre d'exemple et de manière non limitative, on peut citer :
- pour les diluants : le lactose, le dextrose, le sucrose, le mannitol, le sorbitol, la cellulose, la glycérine ;
- pour les lubrifiants : la silice, le talc, l'acide stéarique et ses sels de magnésium et de calcium, le polyéthylène glycol ;
- pour les liants : le silicate d'aluminium et de magnésium, l'amidon, la gélatine, la tragacanthe, la méthylcellulose, la carboxyméthylcellulose de sodium et la polyvinylpyrrolidone.

Parmi les compositions pharmaceutiques selon l'invention, on retiendra plus particulièrement celles qui conviennent pour l'administration par voie orale, parentérale et notamment intraveineuse, per ou transcutanèe, nasale, rectale, perlinguale, oculaire, respiratoire et plus spécifiquement les comprimés simples ou dragéifiés, les comprimés sublinguaux, les gélules, les glossettes, les capsules, les tablettes, les préparations injectables, les aérosols, les gouttes oculaires ou nasales, les suppositoires, les crèmes, pommades ou gels dermiques.

La voie d'administration préférée est la voie orale et les compositions pharmaceutiques correspondantes peuvent permettre la libération instantanée ou différée des principes actifs.

Les compositions pharmaceutiques préférées sont les comprimés.

La posologie est adaptable selon la nature et la sévérité de l'affection la voie d'administration ainsi que l'âge et le poids du patient. Dans les compositions selon l'invention, elle s'échelonne entre 25 et 150 mg pour le β-bloquant et entre 2.5 et 30 mg d'inhibiteur du courant If sinusal en une ou plusieurs prises par 24 heures. De préférence, lorsque l'inhibiteur du courant If sinusal est l'ivabradine la dose d'administration est comprise entre 5 et 7.5 mg deux fois par jour (b.i.d). Lorsque le β-bloquant est l'atenalol la dose d'administration journalière est, de préférence, de 50 mg en une prise.

Enfin, les compositions pharmaceutiques selon l'invention sont utiles pour la fabrication de médicament destiné au traitement de l'angine de poitrine, de l'ischémie et de l'insuffisance cardiaque.

Les exemples de composition ci-dessous sont donnés à titre non limitatif.

Comprimés de ivabradine / atenolol :

**Exemple 1 ;**

| Constituants | Quantité (mg) |
|---|---|
| Ivabradine, chlorydrate | 5 |
| atenolol | 50 |
| silice colloïdale hydrophobe | 0.4 |
| amidon | 6 |
| stéarate de magnésium | 2 |
| cellulose microcristalline | 50 |
| lactose | 86.6 |
| Pour un comprimé terminé à | 200 |

**Exemple 2 :**

| Constituants | Quantité |
|---|---|
| | (mg) |
| Ivabradine, chlorydrate | 7.5 |
| atenolol | 50 |
| silice colloïdale hydrophobe | 0.4 |
| amidon | 6 |
| stéarate de magnésium | 2 |
| cellulose microcristalline | 50 |
| lactose | 84.1 |
| Pour un comprimé terminé à | 200 |

Etude clinique :
Les patients sélectionnés dans cette étude clinique sont des hommes et des femmes de 18 à 75 ans atteints d'angine de poitrine depuis au moins 3 mois et traités par des β-bloquants tel que l'atenolol (50mg o.d.) ou tout autre β-bloquant à un dosage équivalent depuis au moins 3 mois. Ces patients présentent en dépit de leur traitement un test positif de tolérance, à l'exercice (ETT) et des symptômes quotidiens d'angine de poitrine.

Cette étude clinique internationale, en double aveugle et en groupe parallèle, a été réalisée sur 889 patients. Les patients recevant tous l'atenolol sont randomisés en deux groupes :
- le premier groupe reçoit, en plus du β-bloquant, de l'ivabradine (chlorhydrate) à la dose de 5mg deux fois par jour pendant deux mois puis à la dose de 7.5mg deux fois par jour pendant encore deux mois ;
- le deuxième groupe reçoit, en plus du β-bloquant, un placebo durant 4 mois.

Les patients sont soumis à un test de tolérance à l'exercice sur tapis roulant (ETT) selon le protocole de Bruce avant la prise d'ivabradine (sous β-bloquant seul) et après 4 mois de traitement par l'association entre de l'ivabradine et l'atenolol. Les principaux paramètres mesurés au cours de l'épreuve d'effort sont :
- la durée totale de l'exercice (TED), le TED inclut le temps d'arrêt du tapis soit une durée d'environ 10 secondes à compter de la demande d'arrêt de l'exercice par le patient;
- le temps pour lequel la douleur « angine de poitrine » oblige le patient à arrêter l'exercice (TLA) ;
- temps d'apparition de la douleur « angine de poitrine » (TAO) ;
- temps d'apparition sur l'enregistrement électrocardiographioue d'un sous décalage segment ST de 1 mm (TST), reflétant l'ischémie, et correspondant au signe électrique de souffrance du muscle cardiaque.

Ces mesures sont effectuées au creux de l'activité de l'association de principes actifs soit 12±1 heures et 24±2 heures après la prise ces principes actifs.

L'association ivabradine / β-bloquant selon l'invention permet une bonne acceptabilité ainsi qu'une bonne sécurité d'emploi selon les données du recueil de la compliance.

Les patients ont en moyenne 60±8 ans. A l'entrée dans l'étude, la pression artérielle moyenne systolique / diastolique et la fréquence cardiaque des patients au repos sont en moyenne respectivement : 127±12 / 78±7 mmHg et 67±7 battements par minute. Après 4 mois de traitement par l'association, la fréquence cardiaque est diminuée de 9±10bpm (β-bloquant + placebo 1±10bpm).

Les améliorations moyennes des différents paramètres mesurés durant le test d'exercice sur tapis roulant entre la valeur mesurée avant le début du traitement par ivabradine et la fin de la période de traitement sont consignés dans le tableau ci-dessous :

**Amélioration des paramètres (en secondes, moyenne ± SD)**

| | β-bloquant + ivabradine | β-bloquant + placebo | Valeur de p |
|---|---|---|---|
| | (n=441) | (n=434) | |
| TED | 24±65 | 8±64 | p< 0.001 |
| TAO | 49±83 | 23±79 | p< 0.001 |
| TLA | 26±66 | 9±64 | p< 0.001 |
| TST | 46±93 | 15±87 | p< 0.001 |

Cette étude montre que l'ivabradine est capable d'améliorer la tolérance à l'exercice de patients recevant déjà une dose standard de β-bloquants.

Le niveau élevé de la compliance (99% chez les patients recevant de l'ivabradine) et le faible pourcentage d'événements indésirables sérieux montrent l'acceptabilité ainsi que la sécurité de l'association ivabradine plus β-bloquant en comparaison au β-bloquant en monothérapie.

## Revendications

1. Association d'un inhibiteur du courant If sinusal et d'un β-bloquant.

2. Association selon la revendication 1, **caractérisée en ce que** l'inhibiteur du courant I_{f} sinusal est l'ivabradine ou 3-{3-[{[(7S) -3,4-diméthoxybicyclo[4.2.0]octa-1,3,5-trién-7-yl]méthyl}(méthyl)amino]propyl}-7,8-di-méthoxy-1,3,4,5-tétrahydro-2*H*-3-benzazépin-2-one, ou un de ses hydrates, formes cristallines ou sels d'addition à un acide pharmaceutiquement acceptable.

3. Association selon l'une des revendications 1 ou 2, **caractérisée en ce que** l'inhibiteur du courant I_{f} sinusal est l'ivabradine ou 3-{3-[{[(7S) -3.4-diméthoxybicyclo[4.2.0]octa-1,3,5-trién-7-yl]méthyl}(méthyl)amino]propyl}-7,8-di-méthoxy-1,3,4,5-tétrahydro-2*H*-3-benzazépin-2-one, chlorhydrate ou un de ses hydrates ou formes cristallines.

4. Association selon l'une des revendications 1 à 3, **caractérisée en ce que** le β-bloquant est un β-bloquant cardio-sélectif.

5. Association selon l'une des revendications 1 à 4, **caractérisée en ce que** le β-bloquant cardio-sélectif est choisi parmi l'acébutolol, aténolol, bétaxolol, bisoprolol, esmolol, metoprolol ou nébivolol.

6. Association selon l'une des revendications 1 à 5, **caractérisée en ce que** le β-bloquant est l'aténolol, ou un de ses hydrates, formes cristallines ou sels d'addition à un acide pharmaceutiquement acceptable.

7. Association selon l'une des revendications 1 à 5, **caractérisée en ce que** le β-bloquant est le bisoprolol, ou un de ses hydrates, formes cristallines ou sels d'addition à un acide pharmaceutiquement acceptable.

8. Association selon l'une des revendications 1 à 5, **caractérisée en ce qu'**elle contient l'ivabradine ou un de ses hydrates, formes cristallines ou sels d'addition à un acide pharmaceutiquement acceptable, et l'aténolol ou un de ses hydrates, formes cristallines ou sels d'addition à un acide pharmaceutiquement acceptable.

9. Association selon l'une des revendications 1 à 5, **caractérisée en ce qu'**elle contient l'ivabradine ou un de ses hydrates, formes cristallines ou sels d'addition à un acide pharmaceutiquement acceptable, et le bisoprolol ou un de ses hydrates, formes cristallines ou sels d'addition à un acide pharmaceutiquement acceptable.

10. Composition pharmaceutique comprenant comme principe actif une association selon l'une des revendications 1 à 9 seul ou en combinaison avec un ou plusieurs excipients pharmaceutiquement acceptables.

11. Composition pharmaceutique selon la revendication 10 utile pour la fabrication d'un médicament pour le traitement de l'angine de poitrine, de l'ischémie et de l'insuffisance cardiaque.

12. Utilisation d'une association selon l'une des revendications 1 à 9 pour la fabrication d'un médicament pour le traitement de l'angine de poitrine, de l'ischémie et de l'insuffisance cardiaque.

13. Utilisation d'une composition pharmaceutique selon l'une des revendications 10 à 11 pour la fabrication d'un médicament pour le traitement de l'angine de poitrine, de l'ischémie et de l'insuffisance cardiaque.
